Europäisches Patentamt

European Patent Office

Office européen des brevets

(19)

(11) Numéro de publication: **0 074 877 B1**

# FASCICULE DE BREVET EUROPEEN

(12)

(45) Date de publication du fascicule du brevet: **01.04.87**

(51) Int. Cl.⁴: **G 01 N 23/08,** G 01 T 1/166

(21) Numéro de dépôt: **82401607.5**

(22) Date de dépôt: **31.08.82**

(54) **Système de contrôle non destructif de la structure interne d'objets.**

(30) Priorité: **10.09.81 FR 8117148**

(43) Date de publication de la demande:
**23.03.83 Bulletin 83/12**

(45) Mention de la délivrance du brevet:
**01.04.87 Bulletin 87/14**

(84) Etats contractants désignés:
**DE GB IT NL**

(56) Documents cités:
**FR-A-2 345 983**
**US-A-3 431 413**
**US-A-4 067 041**

(73) Titulaire: **COMMISSARIAT A L'ENERGIE
ATOMIQUE Etablissement de Caractère
Scientifique Technique et Industriel
31/33, rue de la Fédération
F-75015 Paris (FR)**

(72) Inventeur: **Huet, Jacques
160, Parc des Eaux Vives
F-91120 Palaiseau (FR)**

(74) Mandataire: **Mongrédien, André et al
c/o BREVATOME 25, rue de Ponthieu
F-75008 Paris (FR)**

Courier Press, Leamington Spa, England.

## Description

La présente invention concerne un système de contrôle non destructif de la structure interne d'objets. Elle s'applique particulièrement au contrôle d'objets, métalliques par exemple, dont la qualité de la structure interne joue un rôle particulièrement important; elle peut aussi s'appliquer au contrôle de la structure interne de pièces surmoulées.

On sait réaliser un contrôle non destructif de la structure d'un objet tel que des pièces métalliques par exemple, à l'aide d'ultrasons. Il est souvent nécessaire en effet de connaître la structure interne de certaines pièces pour les caractériser de manière à mieux les façonner. Dans le contrôle par ultrasons, on dirige sur la pièce ou l'objet à contrôler un faisceau ultrasonore et on étudie sur les faisceau ultrasonore réfléchi sur les défauts internes de l'objet par exemple, les variations de l'énergie de ce faisceau réfléchi, de manière à pouvoir caractériser et situer ces défauts. Dans le contrôle par ultrasons, il est très difficile d'obtenir une image précise de la structure interne d'un objet à cause des échos parasites qui apparaissent à la réflexion des faisceaux ultrasonores à l'intérieur de l'objet. On sait aussi, pour l'étude du corps humain utiliser un procédé dit de "tomographie" par rayons X. Selon ce procédé, on reconstitue une image d'une coupe du corps humain ou de l'un de ses organes, par traitement des informations relatives à l'atténuation d'un faisceau de rayons X ayant traversé le corps ou l'organe; le faisceau de rayons X atténué est capté par un ou plusieurs détecteurs qui permettent de mesurer cette atténuation et d'en déduire les densités des tissus traversés. Alors que l'image radiographique traditionnelle met en évidence les atténuations cumulées de rayonnement, l'image tomographique dégage les tissus observés de l'accumulation des ombres portées des organes environnants. Le niveau de résolution atteint permet de déceler de faibles variations de densité des tissus et donc d'améliorer la différenciation entre deux tissus voisins.

Généralement, les appareils de tomographie par rayons X comprennent un ensemble mécanique mobile en rotation dont l'axe passe sensiblement par le centre de la partie du corps ou de l'organe examiné; cet ensemble supporte un générateur de rayons X et un détecteur de rayonnement, eux-mêmes mobiles linéairement dans un plan perpendiculaire à l'axe du système. Le générateur et le détecteur comprennent les diaphragmes qui permettent de définir des tranches d'observation du corps ou de l'organe, de faible épaisseur. Les signaux analogiques du détecteur sont convertis en impulsions numériques, puis traités par un calculateur qui permet de piloter les déplacements circulaires et linéaires, d'acquérir les données de mesure, de reconstruire et de visualiser sur un écran l'image reconstruite et de stocker les éléments de l'image reconstruite dans une mémoire. Généralement, ce calculateur est associé à des unités périphériques complémentaires telles qu'une imprimante qui permet d'effectuer un relevé de la densité des tissus traversés par les rayons X. Les générateurs de rayons X utilisés dans les appareils de tomographie du corps humain ont une tension qui n'excède pas 150 volts du fait de la faible densité des tissus étudiés. En effet, les phénomènes d'interaction photons-matière, résultent uniquement de l'absorption photoélectrique de la diffusion de rayons X dont la combinaison conduit à une atténuation exponentielle de ceux-ci. Des appareils de ce type sont décrits dans le brevet français N° 2 345 983.

Pour faire des examens tomographiques plus rapides et afin de ne pas exposer le corps humain aux rayons X pendant de trop longues durées et pour améliorer la qualité des images tomographiques obtenues, les tomographes actuels présentent un grand nombre de détecteurs, de telle sorte que le faisceau de rayons X issu du générateur présente la forme d'un éventail qui s'étend du générateur vers les détecteurs. Il en résulte qu'avec ces appareils il n'est plus nécessaire de déplacer linéairement l'ensemble générateur-détecteur pour obtenir l'image d'une coupe entière du corps humain. Un appareil de ce type est décrit dans le brevet US N° 4 047 041.

Le système de l'invention s'apparente aux appareils tomographiques par rayons X dans lesquels l'ensemble générateur-détecteur est fixe par rapport à un repère de référence. Ce système a pour principal avantage d'éviter de déplacer l'ensemble générateur-détecteur qui comporte, notamment dans le cas des détecteurs multicellulaires, un grand nombre de liaisons électriques qui permettent de prélever les signaux de mesure sur les cellules de détection; ces liaisons entraînent en effet de difficiles problèmes de connexion. Le générateur et le détecteur étant immobiles, il est possible d'atteindre pour ceux-ci des dimensions importantes qui ne peuvent être atteintes avec les systèmes dans lesquels l'ensemble générateur-détecteur est mobile en rotation.

Le système de contrôle non destructif de structure d'objets, conforme à l'invention a pour but de contrôler la structure interne d'objets ou de pièces, telles que des pièces métalliques par exemple, de façon beaucoup plus précise qu'avec les appareils à ultrasons.

L'invention a pour objet un système de contrôle non destructif de la structure interne d'objets, comprenant un support d'objets à contrôler et un ensemble fixe constitué par un générateur de rayons X capable d'émettre un faisceau incident de rayons X en direction de l'objet et par des moyens de détection du faisceau atténué de rayons X issu de l'objet dans la direction du faisceau incident, ces moyens de détection étant solidaires du générateur et fournissant au moins un signal dont l'amplitude est proportionnelle à l'intensité du faisceau atténué, caractérisé en ce qu'il comprend des moyens de commande de déplacements successifs du support par rapport à un repère fixe de référence, ces moyens de commande comprenant des moyens pour déplacer linéairement le support, parallèlement à un axe qui est contenu dans un plan de coupe P de l'objet et qui est perpendiculaire au faisceau incident, des moyens pour déplacer linéairement le support, parallèlement à un axe qui est perpendiculaire au plan de coupe, et des moyens pour faire tourner le support autour de l'axe perpendiculaire au plan de coupe, ce système comprenant aussi des moyens de traitement des signaux issus des moyens de détection, ces moyens de traitement comprenant:

— des moyens d'amplification des signaux issus des moyens de détection;

— des moyens d'intégration du signal reliés aux moyens d'amplification, ces moyens d'intégration étant synchronisés sur les déplacements successifs du support, de sorte que la durée d'intégration corresponde à l'intervalle de temps séparant deux déplacements linéaires successifs du support;

— un convertisseur analogique-numérique relié aux moyens d'intégration pour fournir des valeurs numériques correspondant respectivement aux amplitudes moyennes des signaux intégrés durant les intervalles de temps séparant les déplacements successifs du support;

— des moyens pour mémoriser ces valeurs numériques;

— des moyens pour traiter ces valeurs numériques mémorisées et pour commander, à partir de ces valeurs, un appareil de visualisation de chaque coupe de l'objet,

ce système est caractérisé en ce que les moyens d'amplification des signaux issus des moyens de détection comprennent au moins un premier amplificateur logarithmique recevant le signal de sortie des moyens de détection, des moyens pour obtenir un signal d'amplitude proportionnelle à l'intensité du faisceau de rayons X incidents, un deuxième amplificateur logarithmique recevant ce signal proportionnel, un sommateur recevant les signaux fournis par les premier et deuxième amplificateurs logarithmiques, une sortie de ce sommateur étant reliée aux moyens d'intégration.

Selon une autre caractéristique, les moyens pour traiter les valeurs numériques sont aptes à commander la synchronisation des moyens d'intégration et des moyens de commande des déplacements du support.

Selon une autre caractéristique, les moyens pour commander les déplacements du support comprennent un premier moteur pas à pas agissant sur le déplacement du support parallèlement à l'axe contenu dans le plan de coupe et perpendiculaire au faisceau incident, un deuxième moteur pas à pas pour commander la rotation du support autour de l'axe perpendiculaire au plan de coupe, un troisième moteur pas à pas pour commander le déplacement du support parallèlement à l'axe perpendiculaire au plan de coupe, l'avance de ces premier, deuxième et troisième moteurs étant respectivement contrôlée par des codeurs d'incrément eux-mêmes connectés aux moyens pour traiter les valeurs numériques et pour synchroniser les moyens de commande d'intégration et les moyens de commande de déplacement, et des moyens de repérage et de codage des positions du support par rapport au repère de référence, ces moyens de repérage et de codage étant reliés aux moyens pour traiter les valeurs numériques et pour synchroniser les moyens d'intégration et les moyens de commande de déplacement.

Enfin selon une autre caractéristique, les moyens pour obtenir un signal d'amplitude proportionnelle à l'intensité du faisceau de rayons X incidents, sont constitués par une chambre d'ionisation.

Les caractéristiques et avantages de l'invention ressortiront mieux de la description qui va suivre, donnée en référence aux dessins annexés, dans lesquels:

— la figure 1 est un schéma qui représente les déplacements du support d'objet, dans le système de l'invention;

— la figure 2 est un schéma qui permet de mieux comprendre comment sont réalisées des coupes de l'objet avec le système de l'invention;

— la figure 3 représente schématiquement un système de contrôle conforme à l'invention;

— la figure 4 représente certains signaux caractéristiques qui apparaissent dans le système de l'invention.

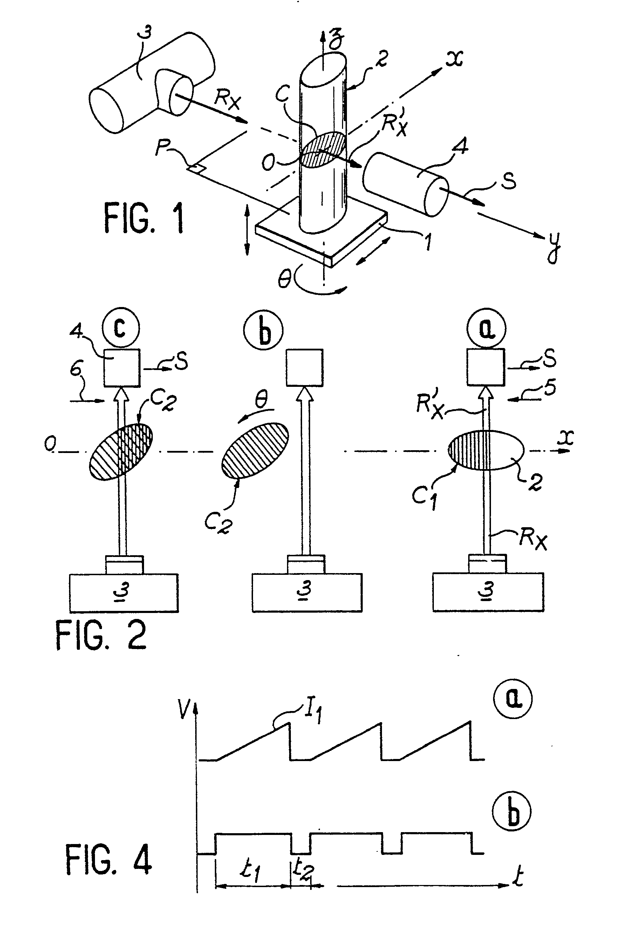

La figure 1 est un schéma qui permet de mieux comprendre le principe de fonctionnement du système de l'invention. Ce système comprend un support 1 de l'objet 2; cet objet peut être une pièce métallique de forme cylindrique, par exemple; le système comprend aussi un ensemble constitué par un générateur 3 de rayons X, capable d'émettre un faisceau incident RX en direction de l'objet 2; cet ensemble comprend également des moyens de détection 4 à une ou plusieurs cellules de détection, qui fournit au moins un signal de sortie S dont l'amplitude est proportionnelle à l'intensité du faisceau atténué de rayons X R'X issu de l'objet. Cet ensemble associé avec le support 1 et à des moyens de traitement (non représentés) des signaux S, permet, comme on le verra plus loin en détail, d'obtenir l'image d'une coupe C de l'objet 2, dans un plan P. Le générateur 3 et les moyens de détection 4 sont rendus solidaires par des moyens non représentés sur la figure et sont fixes par rapport à un repère de référence tel que Oxyz par exemple. La coupe C est située dans un plan P qui contient le faisceau incident RX et le faisceau atténué R'x issu de l'objet, ces faisceaux se propageant selon l'axe OY qui passe par les diaphragmes (non représentés) du générateur et du détecteur. Le support 1 peut être déplacé, comme on le verra plus loin en détail, parallèlement à un axe Ox situé dans le plan de coupe et perpendiculaire au faisceau incident RX; il peut aussi être déplacé parallèlement à l'axe Oz perpendiculaire au plan de coupe; enfin, ce support peut tourner d'un angle θ autour de l'axe Oz perpendiculaire au plan de coupe (θ étant compris entre 0 et 360°).

La figure 2 permet de mieux comprendre comment il est possible d'obtenir une image d'une coupé C de l'objet, grâce au système de l'invention. Sur cette figure, on a représenté schématiquement une partie du système de l'invention, pour différentes positions a, b, et c de l'objet par rapport à l'ensemble générateur 3-moyens de détection 4.

Dans la position *a*, on suppose que l'objet est déplacé, grâce à son support, dans le sens de la flèche 5, parallèlement à la direction OX et perpendiculairement à la direction du faisceau incident RX. Au cours de ce déplacement, la coupe de l'objet est représentée schématiquement en $C_1$.

Pour chacune des positions du support par rapport à l'ensemble générateur-moyens de détection, les signaux S issus des moyens de détection, sont mémorisés, après une conversion analogique-numérique, comme on le verra plus loin en détail.

Dans le position *b*, on suppose que la totalité de la coupe $C_2$ de l'objet, a été balayée lorsque celui-ci était déplacé dans le sens de la flèche 5; on suppose aussi que l'on a fait subir à l'objet une rotation θ autour de l'axe z (figure 1) perpendiculaire au plan de coupe. L'objet est alors déplacé à nouveau dans la direction Ox, mais en sens inverse, tel que représenté par la flèche 6. La coupe de l'objet est ici représentée en $C_2$. Comme précédemment, les signaux issus des moyens de détection pour chaque position de l'objet et de son support par rapport à l'ensemble générateur-moyens de détection, sont mémorisés après conversion analogique-numérique, comme on le verra plus loin en détail.

On peut alors faire subir à l'objet une nouvelle rotation autour de l'axe OZ perpendiculaire au plan de coupe, de manière à obtenir des valeurs supplémentaires de l'intensité du faisceau atténué R'x issue de l'objet pour permettre d'obtenir une meilleure image de la coupe C de cet objet.

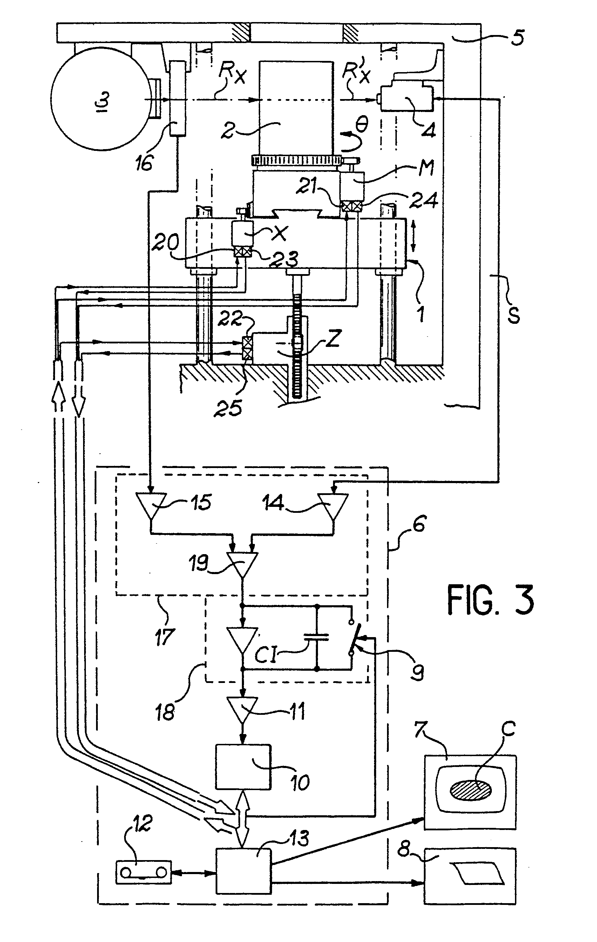

La figure 3 représente de manière plus détaillée un système de contrôle conforme à l'invention. Ce système comprend, comme on l'a indiqué plus haut, un ensemble constitué par un générateur 3 de rayons X, capable d'émettre un faisceau incident RX de rayons X, en direction de l'objet 2 et des moyens de détection 4, constitués par une ou plusieurs cellules de détection qui reçoivent le faisceau atténué R'X de rayons X issus de l'objet. Le générateur 3 et les moyens de détection 4 sont rendus solidaires par un bâti 5. Le système comprend aussi des moyens de commande des déplacements successifs du support 1 par rapport au repère fixe de référence Ox, y, z, lié à l'ensemble générateur-moyens de détection. Ces moyens de commande des déplacements seront décrits plus loin en détail; ils permettent, en fait, au faisceau incident RX de balayer au moins un plan de coupe P de l'objet, comme on l'a expliqué précédemment en regard de la figure 2. Le système comporte également des moyens 6 de traitement des signaux issus des moyens de détection 4, au cours du déplacement du support, pour visualiser sur un appareil de visualisation au moins une coupe C d'objet, comme on le verra plus loin en détail.

Les moyens 6 de traitement des signaux issus des moyens de détection 4, au cours des déplacements du support 1 comprennent des moyens d'amplification 17 des signaux issus des moyens de détection 4, et des moyens d'intégration 18 qui sont reliés à la sortie des moyens d'amplification 17. Ces moyens d'intégration sont constitués par exemple par un circuit à amplificateur opérationnel dont il est possible de court-circuiter le condensateur d'intégration CI par un interrupteur 9 à commande électronique par exemple. Les moyens d'intégration sont synchronisés, comme on le verra plus loin en détail, sur les déplacements successifs du support 1, de sorte que la durée d'intégration corresponde à l'intervalle de temps séparant deux déplacements linéaires successifs du support.

Les moyens 13 de traitement des signaux comprennent également un convertisseur analogique-numérique 10 relié à la sortie des moyens d'intégration 18, par exemple par l'intermédiaire d'un amplificateur 11. Ce convertisseur analogique-numérique fournit des valeurs numériques qui correspondent respectivement aux amplitudes moyennes des signaux intégrés durant les intervalles de temps qui séparent les déplacements successifs du support. Enfin, ces moyens de traitement comprennent des moyens 12 pour mémoriser les valeurs numériques fournies par le convertisseur 10 et des moyens 13 pour traiter ces valeurs numériques mémorisées et pour commander, à partir de ces valeurs mémorisées, un appareil de visualisation de chaque coupe de l'objet. Les moyens de mémorisation 12 peuvent être constitués par exemple, par une unité à bande magnétique, tandis que les moyens de traitement 13 sont constitués par un calculateur éventuellement équipé d'un opérateur cable rapide. L'appareil de visualisation peut être constitué, comme on l'a mentionné plus haut, soit par un appareil de visualisation à tube cathodique 7, soit par une imprimante 8.

Les moyens 17 d'amplification des signaux S issus des moyens de détection 4 comprennent un premier amplificateur logarithmique 14 qui reçoit les signaux S et un deuxième amplificateur logarithmique 15 qui est relié à la sortie de moyens 16 qui permettent d'obtenir un signal dont l'amplitude est proportionnelle à l'intensité du faisceau de rayons X incidents. Ces moyens 16 sont constitués par une chambre d'ionisation qui n'est pas représentée en détail sur la figure. Enfin, les moyens d'amplification 17 comprennent un sommateur 19 dont les entrées sont reliées aux sorties des amplificateurs logarithmiques 14, 15. La sortie de ce sommateur délivre donc un signal dont l'amplitude est proportionnelle au produit de l'intensité du faisceau de rayons X incidents (intensité constante) et de l'intensité du faisceau de rayons X issus de l'objet. La sortie de ce sommateur est reliée aux moyens d'intégration 18 qui reçoivent en fait, un signal dont l'amplitude est proportionnelle à l'intensité du faisceau R'X de rayons X issus de l'objet 1.

Les moyens 13 qui permettent de traiter les valeurs numériques fournies par le convertisseur analogique-numérique 10 permettent également de commander la synchronisation des moyens d'intégration 18 et des moyens de commande des déplacements du support qui vont maintenant être décrits de manière plus détaillée.

Ces moyens de commande de déplacements du support comprennent un premier moteur X de type pas à pas, qui agit sur le déplacement du support parallèlement à l'axe Ox contenu dans le plan de coupe P (figure 1) et perpendiculaire au faisceau incident RX. Ces moyens comprennent aussi un deuxième moteur M de type pas à pas, pour commander la rotation du support autour de l'axe Ox perpendiculaire au plan de coupe P. Enfin, un troisième moteur Z de type pas à pas, permet de commander le déplacement du support parallèlement à l'axe Oz perpendiculaire au plan de coupe P. Enfin, ces moteurs commandent, par exemple, le déplacement de crémaillères liées au support 1. Ces moteurs de type pas à pas sont contrôlés respectivement par des codeurs d'incrément 20, 21, 22, connus dans l'état de la technique, ces codeurs d'incrément étant eux-mêmes connectés aux moyens 13 de traitement de valeurs numériques. En fait, le moteur X permet de commander le déplacement du support de manière à balayer le plan de coupe comme le montre la figure 2(a); le moteur M permet de faire subir une rotation au support 1, comme le montre la figure 2(b); enfin, le moteur Z permet de définir un autre plan de coupe. Les positions du support peuvent être repérées par des moyens de repérage et de codage 23, 24, 25, qui sont reliés aux moyens de traitement 13 et qui sont bien connus dans l'état de la technique. En fait, ces moyens de repérage et de codage permettent d'informer à chaque instant les moyens de traitement 13 sur la position du support par rapport au repère fixe de référence Oxyz.

La figure 4 est un diagramme qui représente en

(a) les signaux de sortie des moyens d'intégration 18, au cours des déplacements du support 1 et, en

(b) les signaux de commande de remise à zéro appliqués à l'interrupteur 9 des moyens d'intégration 18, par les moyens de traitement 13. En fait, les moyens de traitement 13 permettent de commander la synchronisation des moyens d'intégration et des moyens de déplacement du support 1. Cette synchronisation est telle que l'interrupteur 9 est ouvert entre deux déplacements successifs du support pour permettre, entre ces deux déplacements successifs, une intégration du signal de sortie des moyens de détection 4. Sur la figure, $t_1$ représente l'intervalle de temps entre deux déplacements successifs du support, tandis que $t_2$

représente la durée d'un déplacement. L'amplitude moyenne d'un signal d'intégration tel que $I_1$ est transformée en valeur numérique par le convertisseur analogique-numérique 10 de la figure 1. On obtient ainsi, pour les différentes positions de support, des valeurs numériques correspondant aux intensités du rayon X issu de l'objet 2. Ces valeurs numériques sont mémorisées par la bande magnétique 12 et lorsque le balayage de l'objet a été effectué complètement ($\theta = 360°$), ces valeurs numériques sont utilisées pour visualiser la coupe C, après traitement.

Le système qui vient d'être décrit permet de visualiser des coupes d'objets métalliques par exemple, grâce à l'utilisation d'un générateur de rayons X présentant une haute tension supérieure à 200 kV.

Il peut permettre le contrôle de pièces pleines ou creuses telles que des tubes utilisés dans des centrales nucléaires par exemple, ces tubes devant être de structure très homogène. Il peut éventuellement s'appliquer au contrôle de fabrication des éléments combustibles des générateurs nucléaires.

Il est bien évident que dans le système décrit, les moyens utilisés auraient pu être remplacés par des moyens équivalents, sans sortir du cadre de l'invention.

**Revendications**

1. Système de contrôle non destructif de la structure interne d'objets, comprenant un support (1) d'objets à contrôler et un ensemble fixe constitué par un générateur (3) de rayons X capable d'émettre un faisceau incident de rayons X (RX) en direction de l'objet (2) et par des moyens de détection (4) du faisceau atténué de rayons X issu de l'objet dans la direction du faisceau incident, ces moyens de détection étant solidaires du générateur et fournissant au moins un signal (S) dont l'amplitude est proportionnelle à l'intensité du faisceau atténué (R'X'), caractérisé en ce qu'il comprend:

— des moyens de commande de déplacements successifs du support par rapport à un repère fixe de référence, ces moyens de commande comprenant des moyens pour déplacer linéairement le support, parallèlement à un axe (Ox) qui est contenu dans un plan de coupe P de l'objet et qui est perpendiculaire au faisceau incident (RX),

— des moyens pour déplacer linéairement le support, parallèlement à un axe (Oz) qui est perpendiculaire au plan de coupe,

— des moyens pour faire tourner le support autour de l'axe (Oz) perpendiculaire au plan de coupe, ce système comprenant aussi des moyens (6) de traitement des signaux issus des moyens de détection, ces moyens de traitement comprenant:

— des moyens (17) d'amplification des signaux issus des moyens de détection;

— des moyens d'intégration (18) du signal reliés aux moyens d'amplification, ces moyens d'intégration étant synchronisés sur les déplacements successifs du support, de sorte que la durée d'intégration corresponde à l'intervalle de temps séparant deux déplacements linéaires successifs du support;

— un convertisseur analogique-numérique (10) relié aux moyens d'intégration pour fournir des valeurs numériques correspondant respectivement aux amplitudes moyennes des signaux intégrés durant les intervalles de temps séparant les déplacements successifs du support;

— des moyens (12) pour mémoriser ces valeurs numériques;

— des moyens (13) pour traiter ces valeurs .numériques mémorisées et pour commander, à partir de ces valeurs, un appareil de visualisation (7 ou 8) de chaque coupe de l'objet, les moyens (17) d'amplification des signaux issus des moyens de détection (4), comprenant au moins un premier amplificateur logarithmique (14) recevant le signal de sortie des moyens de détection, des moyens (16) pour obtenir un signal d'amplitude proportionnelle à l'intensité du faisceau de rayons X incidents, un deuxième amplificateur logarithmique (15) recevant ce signal proportionnel, un sommateur (19) recevant les signaux fournis par les premier et deuxième amplificateurs logarithmiques, une sortie de ce sommateur étant reliée aux moyens d'intégration.

2. Système selon la revendication 1, caractérisé en ce que les moyens (13) pour traiter les valeurs numériques sont aptes à commander la synchronisation des moyens d'intégration et des moyens de commande des déplacements du support.

3. Système selon la revendication 2, caractérisé en ce que les moyens pour commander les déplacements du support comprennent un premier moteur (X) pas à pas agissant sur le déplacement du support parallèlement à l'axe (Ox) contenu dans le plan de coupe et perpendiculairement au faisceau incident, un deuxième moteur (M) pas à pas pour commander la rotation du support autour de l'axe (Oz) perpendiculaire au plan de coupe, un troisième moteur (Z) pas à pas pour commander le déplacement du support parallèlement à l'axe (Oz) perpendiculaire au plan de coupe, l'avance de ces premier, deuxième et troisième moteurs étant respectivement contrôlée par des codeurs d'incrément, eux-mêmes connectés aux moyens pour traiter les valeurs numériques et pour synchroniser les moyens de commande d'intégration et les moyens de commande de déplacement, et des moyens de repérage et de codage des positions du support par rapport au repère de référence, ces moyens de repérage et de codage étant reliés aux moyens pour traiter les valeurs numériques et pour synchroniser les moyens d'intégration et les moyens de commande de déplacement.

4. Système selon la revendication 3, caractérisé en ce que les moyens (16) pour obtenir un signal d'amplitude proportionnelle à l'intensité du faisceau de rayons X incidents, sont constitués par une chambre d'ionisation.

**Patentansprüche**

1. System für die nicht-destruktive Prüfung der Innenstruktur von Objekten, enthaltend einen Träger (1) für die zu prüfenden Objekte und eine feste Einrichtung, die gebildet ist von einem Röntgenstrahlengenerator (3), der in der Lage ist, ein Röntgenstrahlenbündel (RX) in Richtung des Objekts (2) abzugeben, und von einer Detektoreinrichtung (4) für das gedämpfte Röntgenstrahlenbündel, das von dem Objekt in Richtung des einfallenden Bündels abgegeben wird, wobei die Detektoreinrichtung fest mit dem Generator verbunden ist und wenigstens ein Signal (S) abgibt, dessen Amplitude proportional der Intensität des gedämpften Strahlenbündels (R'X') ist, dadurch gekennzeichnet, daß es enthält:

eine Einrichtung zum Steuern aufeinanderfolgender Verschiebungen des Trägers gegenüber einer festen Bezugsmarkierung, wobei diese Steuereinrichtung eine Einrichtung zum linearen Verschieben des Trägers parallel zu einer Achse (Ox) aufweist, die in einer Schnittebene P des Objekts enthalten ist und die senkrecht zum einfallenden Bündel (RX) ist,

eine Einrichtung zum linearen Verschieben des Trägers parallel zu einer Achse (Oz), die senkrecht zu der Schnittebene ist,

eine Einrichtung zum Drehen des Trägers um die zur Schnittebene senkrechte Achse (Oz), wobei das System weiterhin eine Einrichtung (6) zum Verarbeiten der von der Detektoreinrichtung abgegebenen Signale enthält, wobei diese Verarbeitungseinrichtung enthält:

eine Einrichtung (17) zur Verstärkung der von der Detektoreinrichtung abgegebenen Signale;

eine Signalintegrationseinrichtung (18), die mit der Verstärkungseinrichtung verbunden ist, wobei die Integrationseinrichtung mit den aufeinanderfolgenden Verschiebungen des Trägers so synchronisiert ist, daß die Integrationsdauer dem Zeitintervall entspricht, das zwei aufeinanderfolgende Verschiebungen des Trägers voneinander trennt;

einen Analog/Digital-Wandler (10),der mit der Integrationseinrichtung verbunden ist, um Digitalwerte entsprechend jeweils den mittleren Amplituden der Signale zu liefern, die während der Zeitintervalle integriert werden, die die aufeinanderfolgenden Verschiebungen des Trägers voneinander trennen;

eine Einrichtung (12) zum Speichern der Digitalwerte;

eine Einrichtung (13) zum Verarbeiten der gespeicherten Digitalwerte und zum Steuern einer Einrichtung (7 oder 8) zum Anzeigen eines jeden Schnittes des Objekts anhand dieser Werte, wobei die Verstärkungseinrichtung (17) für die von der Detektoreinrichtung (4) abgegebenen Signale enthält: wenigstens einen ersten logarithmischen Verstärker (14), der das Ausgangssignal der Detektoreinrichtung erhält, eine Einrichtung (16) zum Erzeugen eines Signals einer Amplitude, die proportional zur Intensität des einfallenden Röntgenstrahlenbündels ist, einen zweiten logarithmischen Verstärker (15), der dieses proportionale Signal erhält, einen Summierer (19), der die von den ersten und zweiten logarithmischen Verstärkern gelieferten Signale erhält, wobei ein Ausgang dieses Summierers mit der Integrationseinrichtung verbunden ist.

2. System nach Anspruch 1, dadurch gekennzeichnet, daß die Einrichtung (13) zum Verarbeiten der digitalen Werte dazu eingerichtet ist, die Synchronisation der Integrationseinrichtung und der Einrichtung zum Steuern der Trägerverschiebungen zu steuern.

3. System nach Anspruch 2, dadurch gekennzeichnet, daß die Einrichtung zum Steuern der Trägerverschiebungen enthält: einen ersten Schrittmotor (X), der auf die Verschiebung des Trägers parallel zur Achse (Ox), die in der Schnittebene enthalten ist und senkrecht zum einfallenden Strahlenbündel liegt, einwirkt, einen zweiten Schrittmotor (M), der die Drehung des Trägers um die zur Schnittebene senkrechte Achse (Oz) steuert, einen dritten Schrittmotor (Z), der die Verschiebung des Trägers parallel zu der zur Schnittebene senkrechten Achse (Oz) steuert, wobei der Antrieb der ersten, zweiten und dritten Motoren jeweils durch Schrittkodierer gesteuert ist, die ihrerseits mit der Einrichtung zum Verarbeiten der Digitalwerte und zum Synchronisieren der Integrationssteuereinrichtung und mit der Einrichtung zum Steuern der Verschiebung verbunden sind, und daß Einrichtungen zur Bezugsmarkierung und zur Kodierung der Stellungen des Trägers gegenüber der Bezugsmarkierung vorgesehen sind, wobei diese Einrichtung zur Bezugsmarkierung und zur Kodierung mit den Einrichtungen zum Verarbeiten der digitalenWerte und zum Synchronisieren der Integrationseinrichtung und der Einrichtung zur Steuerung der Verschiebung verbunden ist.

4. System nach Anspruch 3, dadurch gekennzeichnet, daß die Einrichtung (16) zum Erzeugen eines Signals einer Amplitude, die proportional zur Intensität der einfallenden Röntgenstrahlen ist, von einer Ionisationskammer gebildet ist.

## Claims

1. System for the non-destructive monitoring of the internal structure of objects, comprising a support (1) for objects to be monitored and a fixed assembly which comprises an X-ray generator (3) capable of emitting an incident beam of X-rays (RX) in the direction of the object (2) and means (4) for detecting the attenuated beam of X-rays which emerges from the object in the direction of the incident beam, these detecting means forming a solid unit with the generator and supplying at least one signal (S), the amplitude of which is proportional to the intensity of the attenuated beam (R'X'), characterized in that it comprises:

means for controlling successive displacements of the support in relation to a fixed reference mark, these controlling means comprising means for displacing the support linearly, parallel to an axis (Ox) which is contained within a sectional plane (P) of the object and which is perpendicular to the incident beam (RX),

means for displacing the support linearly, parallel to an axis (Oz) which is perpendicular to the sectional plane,

means for causing the support to turn about the axis (Oz) perpendicular to the sectional plane, this system further comprising means (6) for processing the signals emerging from the detecting means, these processing means comprising:

means (17) for amplifying the signals emerging from the detecting means;

means (18) for integrating the signal, which are connected to the amplifying means, these integrating means being synchronized with respect to the successive displacements of the support, in such a manner that the duration of integration corresponds to the time interval separating two successive linear displacements of the support;

an analogue-digital converter (10) connected to the integrating means to supply digital values corresponding respectively to the average amplitudes of the signals integrated during the time intervals separating the successive displacements of the support;

means (12) for memorizing these digital values;

means (13) for processing these memorized digital values and for controlling, with reference to these values, an apparatus (7 or 8) for visualizing each section of the object, the means (17) for amplifying the signals emerging from the detecting means (4) comprising at least one first logarithmic amplifier (14) receiving the output signal of the detecting means, means (16) for obtaining a signal of amplitude proportional to the intensity of the beam of incident X-rays, a second logarithmic amplifier (15) receiving this proportional signal, a summer (19) receiving the signals supplied by the first and second logarithmic amplifiers, one output of this summer being connected to the integrating means.

2. System according to Claim 1, characterized in that the means (13) for processing the digital values are capable of controlling the synchronization of the integrating means and of the means for controlling the displacements of the support.

3. System according to Claim 2, characterized in that the means for controlling the displacements of the support comprise a first stepping motor (X) acting on the displacement of the support parallel to the axis (Ox) contained within the sectional plane and perpendicular to the incident beam, a second stepping motor (M) to control the rotation of the support about the axis (Oz) perpendicular to the sectional plane, a third stepping motor (Z) to control the displacement of the support parallel to the axis (Oz) perpendicular to the sectional plane, the advance of these first, second and third motors being respectively controlled by increment coders, which are themselves connected to the means for processing the digital values and for synchronizing the integration control means and the displacement control means, and means for registering and coding the positions of the support in relation to the reference mark, these registering and coding means being connected to the means for processing the digital values and for synchronizing the integrating means and the displacement control means.

4. System according to Claim 3, characterized in that the means (16) for obtaining a signal of amplitude proportional to the intensity of the beam of incident X-rays comprise an ionization chamber.

**0 074 877**

FIG. 1

FIG. 2

FIG. 4

1

FIG. 3